Europäisches Patentamt

European Patent Office

Office européen des brevets

(11) Numéro de publication: **0 230 794**
**A1**

## (12) DEMANDE DE BREVET EUROPEEN

(21) Numéro de dépôt: **86400166.4**

(51) Int. Cl.⁴: **A61B 5/04**

(22) Date de dépôt: **28.01.86**

(43) Date de publication de la demande:
**05.08.87 Bulletin 87/32**

(84) Etats contractants désignés:
**AT BE CH DE GB IT LI LU NL SE**

(71) Demandeur: **Ascher, Gilles**
**20bis, Boulevard du Général Leclerc**
**F-92200 Neuilly(FR)**
Demandeur: **Fournial, Jean-François**
**La Sauvagère Route de Parinier**
**F-72560 Change(FR)**

(72) Inventeur: **Ascher, Gilles**
**20bis, Boulevard du Général Leclerc**
**F-92200 Neuilly(FR)**
Inventeur: **Fournial, Jean-François**
**La Sauvagère Route de Parinier**
**F-72560 Change(FR)**

(74) Mandataire: **Rodhain, Claude**
**Cabinet Claude Rodhain 30, rue la Boétie**
**F-75008 Paris(FR)**

(54) **Dispositif protecteur d'électrode pour électrocardiogramme enregistré en mode ambulatoire pour la méthode de Holter.**

(57) Ce dispositif comprend un organe de forme de cuvette ou de coupelle ayant un fond (13), une partie formant une paroi latérale périphérique (14) présentant une arête (15) définissant un plan, et une collerette (16) solidaire de ladite arête et présentant à l'état non contraint une configuration plane, ladite collerette étant revêtue d'un adhésif par contact sur sa surface opposée à celle hors de laquelle s'étend ladite cuvette.

FIG.2

## Dispositif protecteur d'électrode pour électrocardiogramme enregistré en mode ambulatoire par la méthode de Holter.

La présente invention est relative aux accessoires utilisés pour pratiquer des électrocardiogrammes en monitoring ou enregistrés en mode ambulatoire, suivant la méthode de Holter (E.C.G.) qui consiste à pratiquer sur un patient l'enregistrement permanent des rythmes cardiaques sur une durée de 24 heures.

Dans la mise en oeuvre de ce procédé on applique directement sur le corps du patient plusieurs électrodes qui constituent des capteurs reliés à un appareil enregistreur portable qui enregistre en continu toutes les variations des pulsations du coeur au cours d'une journée complète d'activité normale du patient.

Les électrodes du type jetable sont constituées par un organe métallique dont la partie inférieure présente habituellement la forme d'un disque destiné à être appliqué sur la peau du patient, ce disque présentant sur sa face opposée un téton pourvu d'une gorge radiale circulaire dans laquelle est fixée une extrémité d'un conducteur souple qui est relié à l'appareil enregistreur.

La partie de l'électrode en forme de disque est recouverte par un disque de diamètre plus grand en une matière mousse souple à travers une ouverture centrale duquel dépasse le téton formant borne. Ce disque est habituellement pourvu d'un adhésif sur la périphérie de sa face inférieure afin de maintenir l'électrode fixée sur la peau du patient.

Le conducteur est normalement fixé sur le téton de l'électrode au moyen d'une légère pince métallique soit en forme de bague fendue soit en forme de ciseau.

Il importe bien entendu qu'au cours de la période pendant laquelle le patient doit porter les électrodes le ou les conducteurs reliés à chaque électrode ne subissent pas de déplacements intempestifs dûs à des tractions excercées sur eux par inadvertance. De même il importe que l'électrode elle-même ne soit pas exposée à des chocs ou à des arrachements pouvant être provoqués par des frottements accidentels.

L'expérience a en effet démontré que ces électrodes ainsi que les conducteurs qui leur sont reliés se trouvaient soumis à diverses sollicitations accidentelles susceptibles de les déplacer, voire de les arracher.

On a constaté que ces chocs, frottements ou déplacements sont générateurs de tensions parasites qui se trouvent de fait enregistrées sur le diagramme de l'appareil enregistreur et gênent considérablement la lecture de celui-ci.

En effet cette lecture peut être effectuée au moyen d'appareils automatiques qui ne sont pas capables de différencier les tensions parasites accidentelles et de les éliminer pour fournir une courbe épurée. Par ailleurs, une lecture directe par le médecin, qui constitue une opération extrêmement longue est également rendue très difficile en raison de ces tensions parasites.

Pour éviter ceci on a utilisé jusqu'à présent un système simple consistant à immobiliser le ou les conducteurs reliés à l'électrode en les fixant sur la peau du patient à une certaine distance de l'électrode au moyen de fragments d'un ruban adhésif quelconque approprié, l'électrode restant fixée grâce aux propriétés adhésives du disque en mousse qui la maintient sur le corps du patient, ce qui est peu efficace et en outre gênant.

L'invention a pour but de remédier à ces artéfacts qui sont susceptibles de masquer des éléments pathologiquement révélateurs de la courbe enregistrée en réalisant un dispositif adapté pour protéger à la fois l'électrode et les conducteurs qui lui sont reliés.

Elle a pour objet à cet effet un dispositif protecteur d'électrode destiné à être utilisé pour la mise en oeuvre de la méthode de Holter, caractérisé en ce qu'il comprend un organe en forme de cuvette ou de coupelle ayant une partie formant une paroi latérale périphérique présentant une arête définissant un plan, et une collerette so lidaire de ladite arête et présentant à l'état non contraint une configuration plane, ladite collerette étant revêtue d'un adhésif par contact sur sa surface opposée à celle hors de laquelle s'étend ladite cuvette.

Suivant une autre caractéristique de l'invention, ledit organe est réalisé en une matière plastique souple déformable et transparente.

De préférence ladite collerette est venue de matière, de fabrication, avec ladite cuvette ou coupelle.

Suivant un mode de réalisation, ledit organe est réalisé par déformation à chaud.

Bien entendu on comprend que ledit organe peut être également formé par un procédé de moulage classique.

D'autres caractéristiques et avantages de l'invention apparaitront au cours de la description qui va suivre faite en se référant au dessin annexé donné uniquement à titre d'exemple et dans lequel:

la Fig.1 montre schématiquement un mode de fixation des conducteurs reliés à une électrode, suivant la technique antérieure;

la Fig.2 est une vue en élévation et en section transversale du dispositif protecteur suivant l'invention posé par-dessus une électrode;

la Fig.3 est une vue en perspective éclatée montrant l'agencement d'un conducteur relié à l'électrode avant la pose du dispositif protecteur suivant l'invention.

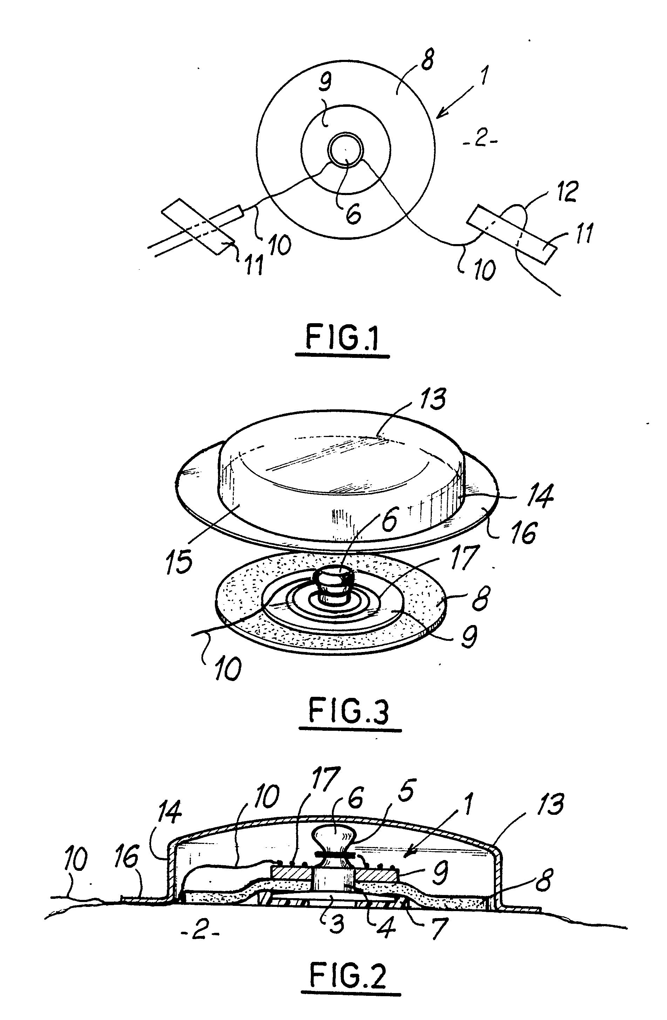

En se référant à la Fig.1, une électrode désignée dans son ensemble par la référence 1 est fixée directement sur la peau 2 d'un patient, cette électrode de configuration classique ayant dans son ensemble une forme analogue à celle de la partie mâle d'un bouton-pression.

Comme connu en soi, cette électrode 1 comprend essentiellement un disque 3 ayant une face inférieure plane, et comportant sur sa face opposée un téton 4 présentant une gorge périphérique 5 et une tête 6. Sur la périphérie du disque 3 est fixée une couronne 7 en une matière plasti que mousse imprégnée d'un liquide conducteur et un disque 8 également en une matière mousse et présentant un trou central est adapté sur le téton 4 de façon que celui-ci s'étende à travers son trou central.

Le disque 8 en matière mousse est souple et présente sur sa face opposée à la saillie de l'arête du téton 4 un revêtement d'un adhésif par contact destiné à adhérer sur la peau du patient.

Une rondelle 9 en une matière relativement rigide comporte un trou central adapté pour que la rondelle puisse être enfoncée à force sur le téton 4, afin de maintenir le disque 8 serré contre la face supérieure du disque 3 de l'électrode.

En se reportant à la Fig.1, le conducteur souple 10 relié à l'électrode dans la gorge 5 de celle-ci était jusqu'à présent maintenu sur la peau du patient au moyen de fragments 11 d'un ruban adhésif approprié, par exemple un ruban adhésif du type "Scotch" ou du type "Sparadrap".

Malgré qu'il soit habituellement prévu que le conducteur 10 puisse décrire une boucle comme représenté en 12 afin de lui assurer un certain jeu dans le cas où une traction serait effectuée par inadvertance sur son brin libre, les déplacements fortuits, les frottements auxquels l'électrode elle-même etait exposee donnaient naissance à des tensions parasites extrêmement nuisibles à la lecture de l'enregistrement effectue, soit par des moyens automatiques soit par la lecture directe par un spécialiste.

Pour pallier cet inconvénient, l'invention fournit un dispositif de protection et de securite constitué, comme représenté aux Fig.2 et 3, par un organe en forme de cuvette ou de coupelle ayant un fond 13 qui est avantageusement bombé et presente une partie de paroi périphérique 14 ayant une arête periphérique 15 definissant un plan, et une collerette radiale 16 solidaire de l'arête 15 et contenue dans le même plan.

La surface de la collerette 16 opposée à la saillie formée par la cuvette 13, 14, est revêtue d'un adhésif par contact afin de maintenir le dispositif collé sur la peau du patient.

Le conducteur 10 qui, suivant l'exemple représenté est fixé dans la gorge 5 du téton 4 au moyen d'une pince élastique est de préférence enroulé autour du téton, pour former une spirale 17 posée librement sur la rondelle 9, le brin sortant du conducteur 10 étant serré entre la surface inférieure de la collerette 16 revêtue d'adhésif et la peau du patient.

On comprend que la spirale 17 (ou autre forme d'enroulement) forme un ressort qui, en cas de traction exercée fortuitement sur le conducteur 10, permet à celui-ci de jouer autour du téton de l'électrode sans répercuter la traction sur celle-ci. Par ailleurs, la tête 6 de l'électrode se trouve ainsi protègée par le fond bombé 13 des chocs et des frottements susceptibles de donner naissance à des tensions parasites nuisibles.

L'électrode est maintenue collée sur la peau du patient par le disque 8 revêtu d'adhésif et il est de préférence prévu un certain jeu entre la périphérie externe du disque 8 et la périphérie interne de la partie de paroi latérale 14 pour tenir compte de l'élasticité de la peau du patient.

Le dispositif de protection suivant l'invention peut avantageusement être réalisé en une matière plastique appropriée souple , par exemple en polycarbonate, et donc déformable pour pouvoir s'adapter sur une partie quelconque du corps, formée d'une seule pièce par exemple par déformation à chaud ou par moulage. La matière utilisée peut également être transparente ou translucide afin de faciliter la mise en place du dispositif de protection selon l'invention, avec un jeu régulier par rapport à l'électrode.

**Revendications**

1-Dispositif protecteur d'électrode pour électrocardiogramme enregistré en monitoring ou en mode ambulatoire par la méthode de Holter, caractérisé en ce qu'il comprend un organe en forme de cuvette ou de coupelle ayant un fond - (13), une partie formant une paroi latérale périphérique (14) présentant une arête (15) définissant un plan, et une collerette (16) solidaire de ladite arête et présentant à l'état non contraint une configuration plane, ladite collerette étant revêtue d'un adhésif par contact sur sa surface opposée à celle hors de laquelle s'étend ladite cuvette (13, 14).

2-Dispositif suivant la revendication 1, caractérisé en ce que ledit organe (13, 14, 16) est en une matière plastique souple déformable et transparente.

3-Dispositif suivant la revendication 2, caractérisé en ce que ladite collerette (16) est venue de matière avec ladite cuvette ou coupelle (13, 14).

4-Dispositif suivant la revendication 3, caractérisé en ce que ledit organe (13, 14, 16) est réalisé par déformation à chaud.

5-Dispositif suivant la revendication 3, caractérisé en ce que ledit organe (13, 14, 16) est réalisé par moulage.

6-Dispositif suivant la revendication 1, caractérisé en ce que ledit organe (13, 14, 16) est réalisé en polycarbonate.

0 230 794

FIG.1

FIG.3

FIG.2

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int Cl 4) |
|---|---|---|---|
| Y | US-A-3 882 853 (GOFMAN et al.) <br> * Figures 1-3; colonne 1, lignes 30-36; colonne 4, ligne 48 - colonne 5, ligne 39 * | 1,2,6 | A 61 B 5/04 |
| A | | 3,5 | |
| | --- | | |
| Y | FR-A-2 359 594 (C.R. BARD INC.) <br> * Figures 1,2,8; page 7, ligne 6 - page 8, ligne 11 * | 1,2,6 | |
| | --- | | |
| A | US-A-3 545 432 (BERMAN) <br> * Figures 1-5; colonne 2, ligne 46 - colonne 3, ligne 48 * | 1,2,5 | |
| | ----- | | |

DOMAINES TECHNIQUES RECHERCHES (Int. Cl.4)

A 61 B
A 61 N

Le present rapport de recherche a ete etabli pour toutes les revendications

| Lieu de la recherche <br> LA HAYE | Date d'achevement de la recherche <br> 26-09-1986 | Examinateur <br> CHEN A.H. |
|---|---|---|